# EUROPEAN PATENT APPLICATION

(11) **EP 4 650 433 A1**
(43) Date of publication of application: **19.11.2025**
(21) Application number: 23916205.0
(22) Date of filing: 14.12.2023
(51) Int. Cl.: C12N 1/14, C12P 13/02

(54) **MYCOSPORINE-GLUTAMINE-CONTAINING EXTRACT**

(30) Priority: 10.01.2023 JP 2023002008
(71) Applicant: Nissui Corporation, Tokyo 105-8676 (JP)
(72) Inventor: NAKAJIMA, Toshiaki, Hachioji-shi, Tokyo 192-0991 (JP); MIZOTA, Ayami, Hachioji-shi, Tokyo 192-0991 (JP)
(74) Representative: Mewburn Ellis LLP
(86) International application number: PCT/JP2023/044829
(87) International publication number: WO 2024/150591

(57) **Abstract**

An object of the present disclosure is to provide a fungal extract which can be industrially produced, and which includes mycosporines (MYCs), especially mycosporine-glutamine (MGn). The present disclosure provides an extract of cultured fungal cells of a fungus, the extract including mycosporines (MYCs), the MYCs including mycosporine-glutamine (MGn). The MYCs may also include one, or two or more, selected from mycosporine-glutaminol-glucoside (MGGnol), mycosporine-glutamicol-glucoside (MGGcol), and mycosporine-glutamic acid (MGa). The fungus used may be a fungus belonging to the genus *Hormonema,* or may especially be *Hormonema macrosporum.*

## Description

### TECHNICAL FIELD

The present disclosure relates to fungal cell extract containing mycosporine-glutamine (MGn), and a method of extracting MGn from a specific fungus.

### BACKGROUND ART

Mycosporine-like amino acids (MAAs) are naturally occurring substances known to efficiently absorb ultraviolet rays such as UV-A and UV-B. MAAs are expected to be applied as a UV-protective agent to be included in sunscreen cosmetics and the like.

Aquatic organisms such as algae and shellfish, as well as microorganisms such as yeasts and molds, are known to produce MAAs, and several ten kinds of such organisms have been found.

For example, in Non-Patent Document 1, fungi, cyanobacteria, and actinomycetes that had been exposed to sunlight were collected, and investigated to determine whether they contain mycosporine (MYC) and MAAs. The document discloses that, as a result, the fungi were found to contain mycosporine-serinol, mycosporine-alanine, mycosporine-serine, mycosporine-glutaminol, mycosporine-glutamicol, mycosporine-glutaminol-glucoside (MGGnol), and mycosporine-glutamicol-glucoside (MGGcol). However, mycosporine-glutamine (MGn) has not been found in fungi.

MGn is a mycosporine that was first discovered in ascospores of a cup fungus (*Pyronema omphalodes*) in 1984. The cup fungus also contained mycosporine-glutamic acid (MGa), normycosporine-glutamine, and mycosporine-glutaminol-glucoside. Mycelia of this fungus could be cultured by a culture method under light irradiation, but MGn was absent in the mycelia (Non-Patent Document 2).

It has been reported that MYC could not be detected in a cultured morel (*Morchella esculenta*) (Non-Patent Document 3), but it has also been reported that there is a difference between wild strains and cultured strains. Specifically, when MYC was investigated for a wild strain and cultured hyphae of a cup fungus, the hymenium of the wild strain (produced by sexual or asexual reproduction) contained about 0.5% MGn, but no MGn could be detected in undifferentiated hyphae obtained by culture under light irradiation. Further, sclerotia of the wild strain also contained 0.26% MGa (Non-Patent Document 2).

MGGnol has also been reported to be contained in carotenoid yeasts and the like, but its content is low when the yeasts are not cultured under light irradiation (Non-Patent Document 4).

*Aureobasidium pullulans,* which is a black yeast of the family Dothioraceae of the order Dothideales, contains MGGnol and MGGcol (Non-Patent Document 1), and it has been reported that MGGnol was produced at 0.34 g/L when the *Aureobasidium pullulans* IDF23 strain, obtained from a natural source, was subjected to liquid culture without light irradiation (Patent Document 1). However, MGn has not been found in *Aureobasidium pullulans.*

For MAAs containing MYCs, there has been a concern about their thermal stability. Mycosporine, mycosporine-glycine, and porphyra-334 have been reported to undergo degradation at 75 to 80°C (Non-Patent Documents 5 and 6).

Fungi belonging to the genus *Hormonema,* which is a member of the family Dothioraceae of the order Dothideales, are known to produce various useful substances such as polymalic acid and antibiotics (Patent Documents 2 to 4). However, production of MAAs by the fungi belonging to the genus *Hormonema,* and by their teleomorphs, that is, the fungi belonging to the genus *Sydowia,* is not known to the best of the inventors' knowledge.

### PRIOR ART DOCUMENTS

### [Patent Documents]

[Patent Document 1] JP 5913988 B
[Patent Document 2] JP 2004-254585 A
[Patent Document 3] JP H3-279379 A
[Patent Document 4] JP H1-215291 A

### [Non-patent Documents]

[Non-Patent Document 1] M. Volkmann et al., FEMS Microbiol. Lett., 255, 286-295 (2006)
[Non-Patent Document 2] Buscot et al., Mycol. Res, 95(6): 752-754 (1991)
[Non-Patent Document 3] Bernillon et al., Phytochemistry, Vol. 23, No. 5, pp. 1083-1087 (1984)
[Non-Patent Document 4] D. Libkind et al., Photochem. Photobiol. Sci., 3, 281-286 (2004)
[Non-Patent Document 5] N. Wada et al., Antioxidants, 4, 603-646 (2015)
[Non-Patent Document 6] M. Yoshiki et al., Food Chemistry, 113, 1127-1132 (2009)
[Non-Patent Document 7] de Hoog et al., Atlas of clinical fungi, 2nd edtion: Utrecht: centraalubureauvoor Schimmelcultures; (2000)
[Non-Patent Document 8] N. A. Yurlava et al., Studies in Mycology., 43, 63-69 (1999)
[Non-Patent Document 9] G. S. de Hoog et al., Antonie van Leeuwenhoek., 65, 41-54 (1994)

### SUMMARY OF THE INVENTION

### PROBLEMS TO BE SOLVED BY THE INVENTION

An object of the present disclosure is to provide a fungal extract which can be industrially produced, and which includes mycosporines (MYCs), especially mycosporine-glutamine (MGn), and to provide a production method therefor.

### MEANS FOR SOLVING THE PROBLEMS

In order to solve the above problem, the inventors of the present disclosure intensively studied to discover that a newly discovered fungus belonging to the genus *Hormonema* produces MGn, which has not been detected in the genus *Aureobasidium* or in the other fungi belonging to the genus *Hormonema* despite the fact that they have conventionally been known to produce MAAs. In particular, the inventors discovered that a large amount of MGn is contained in cultured fungal cells obtained by liquid culture of a fungus belonging to the genus *Hormonema* without light irradiation in a medium containing only glucose and yeast extract. Further, the inventors discovered that an extraction method including heat treatment allows efficient extraction of MYCs including MGn from fungal cells of a fungus belonging to the genus *Hormonema,* to enable production of an MYC-containing fungal cell extract.

Specifically, the present disclosure includes the following inventions. The term "MYCs" may include a single type or a plurality of types of mycosporines.
[1] An extract of cultured fungal cells of a fungus, the extract comprising a mycosporine (MYC),
   the MYC comprising mycosporine-glutamine (MGn).
[2] The extract according to [1], wherein the MYC further comprises one, or two or more, selected from mycosporine-glutaminol-glucoside (MGGnol), mycosporine-glutamicol-glucoside (MGGcol), and mycosporine-glutamic acid (MGa).
[3] The extract according to [1] or [2], wherein the fungus is a fungus belonging to the genus *Hormonema.*
[4] The extract according to [3], wherein the fungus belonging to the genus *Hormonema* is *Hormonema macrosporum.*
[5] The extract according to [4], wherein the *Hormonema macrosporum* is selected from the NSK102 strain (NITE BP-03776), the NSK33 strain (NITE BP-03775), and the NSK6 strain (NITE BP-03774).
[6] The composition according to any one of [1] to [5], wherein MGn is contained at not less than 1% by weight in the entire composition.
[7] The extract according to [2], comprising MGGnol at not less than 30% by weight in the entire composition.
[8] The extract according to [2], comprising MGGcol at not less than 0.5% by weight in the entire composition.
[9] The extract according to any one of [1] to [8], which is a water extract.
[10] A method of producing an MYC-containing fungal cell extract, the method comprising: a culture step of culturing *Hormonema macrosporum;* and an extraction step of extracting a mycosporine (MYC) fraction from cultured fungal cells of the *Hormonema macrosporum;*
   wherein the *Hormonema macrosporum* is selected from the NSK102 strain (NITE BP-03776), the NSK33 strain (NITE BP-03775), and the NSK6 strain (NITE BP-03774).
[11] The production method according to [10], wherein the extract is a polar solvent extract, the method satisfying one or more selected from the following (a), (b), and (c):
   (a) in the extraction step, the MYC fraction is extracted into a polar solvent at not less than 40°C;
   (b) before the extraction step, a heating step of collecting fungal cells from the culture liquid after the culture step, and heating the collected fungal cells, is carried out; and
   (c) in the culture step, the culture is carried out in a medium containing glucose and yeast extract, and/or carried out without light irradiation.
[12] The production method according to [11], wherein the (a) is carried out by heating the culture liquid after the culture step to not less than 50°C.
[13] The production method according to [11], wherein the (b) heating step is carried out by heating under one or more conditions selected from the group consisting of (i) to (iii):
   (i) a temperature of not less than 105°C;
   (ii) a heating time of not less than 2 hours; and
   (iii) a pressure of not more than 0.10 MPa.
[14] The production method according to any one of [10] to [13], wherein the MYC comprises: MGn; and one, or two or more, selected from MGGnol, MGGcol, and MGa.
[15] The production method according to [11], wherein the polar solvent is water.
[16] The production method according to any one of [10] to [15], wherein ethanol and/or butyl alcohol is/are not substantially used as the extraction solvent.

### EFFECT OF THE INVENTION

According to the present disclosure, a fungal extract which can be industrially produced, and which comprises MYCs containing MGn, and a method of producing the extract can be provided. Fungal culture extracts containing MGn are very useful as novel industrial products.

Further, the present disclosure enables extraction of MYCs with high recovery in a short time from fungal cells of *Hormonema macrosporum* that comprise MYCs containing MGn. This can be associated with the fact that heating of the fungal cells of *Hormonema macrosporum* promotes release of MYCs to the outside of the fungal cells, and/or the fact that inactivation of degrading enzymes in the fungal cells occurs to suppress degradation of MYCs, leading to the improvement of the recovered amount. In addition, use of a specific medium in the culture was found to allow low-viscosity culture, by which large-scale production can be easily carried out, and/or to allow the fungal cells to produce a larger amount of MYCs. By this, the amount of MYCs recovered can be expected to be increased. The increased recovery of MYCs allows production of an extract, especially a water extract, containing MYCs at high concentration.

Further, as shown by the present disclosure, MYCs are stable and not degraded by heating or pressurization, so a product containing MYCs can be subjected to sterilization in any step, which is industrially very advantageous.

### MODE FOR CARRYING OUT THE INVENTION

The fungus in the present disclosure is not limited, and any fungus may be used as long as it produces mycosporine-glutamine (MGn). Specific examples of such a fungus that may be used include, but are not limited to, fungi belonging to the genus *Hormonema* that produce mycosporine-glutamine (MGn). Fungi belonging to the genus *Hormonema* can maintain high stability during culture.

Here, the fungi belonging to the genus *Hormonema* include those whose teleomorphs correspond to the genus *Sydowia.* The anamorph of the genus *Sydowia* corresponds to the genus *Hormonema,* and the same fungal strain shows such a difference depending on whether its reproduction occurs sexually or asexually (Non-Patent Documents 7 and 8). Since the fungal cells described as the genus *Sydowia* in the present description also exhibit a yeast-like shape when they are grown by liquid culture, they can be judged as the genus *Hormonema,* which is the anamorph. Therefore, both are described in an inclusive manner (Non-Patent Document 9)

Further, although the fungi whose anamorphs belong to the genus *Aureobasidium* and the fungi whose anamorphs belong to the genus *Hormonema* have a close relationship to each other, these are clearly distinguishable by molecular phylogeny (Non-Patent Document 7).

In the present disclosure, the fungus belonging to the genus *Hormonema* is not limited, and any strain may be used as long as it produces MYCs.

Examples of the fungus belonging to the genus *Hormonema* include *Hormonema macrosporum* and *Hormonema dematioides.* Specific examples of their fungal strains include the NSK102 strain (NITE BP-03776), the NSK33 strain (NITE BP-03775), and the NSK6 strain (NITE BP-03774) of *Hormonema macrosporum.* All of these fungal strains have high storage resistance.

The fungus belonging to the genus *Hormonema* to be used as the extraction source may be of either a single fungal strain or a plurality of fungal strains.

Each of the NSK102 strain (NITE BP-03776), the NSK33 strain (NITE BP-03775), and the NSK6 strain (NITE BP-03774) of *Hormonema macrosporum* is a novel fungus isolated from a natural source. When these fungi were subjected to liquid culture, and the shapes of their cells were observed under a light microscope, yeast-type cells were found. In order to investigate the genetic characteristics of these fungi, the base sequence of the D1/D2 region of 28S rRNA was identified by an ordinary method. Further, the base sequence of the 28S rRNA gene of each *Hormonema macrosporum* strain was subjected to homology search against DB-FU15.0 (Techno Suruga Laboratory, Japan) and international base sequence databases (DDBJ/ENA/GenBank) (search date, July 29, 2022) by BLAST analysis using the analysis software ENKI v3.2 (Techno Suruga Laboratory, Japan). In the analysis, a phylogenetic tree was estimated by the neighbor-joining method. The Kimura-2-parameter was used as a base substitution model, and the reliability of the topology was evaluated by the bootstrap method (1000 replications). As a result, all three strains were identified as *Hormonema macrosporum* (anamorph) or *Sydowia polysopra* (teleomorph).

The NSK102 strain of *Hormonema macrosporum* has been internationally deposited with Patent Microorganisms Depositary, National Institute of Technology and Evaluation (Address: Room 122, 2-5-8 Kazusakamatari, Kisarazu-shi, Chiba, 292-0818 Japan) under the accession No. NITE BP-03776 as of October 20, 2022 under the Budapest Treaty.

The NSK33 strain of *Hormonema macrosporum* has been internationally deposited with Patent Microorganisms Depositary, National Institute of Technology and Evaluation (Address: Room 122, 2-5-8 Kazusakamatari, Kisarazu-shi, Chiba, 292-0818 Japan) under the accession No. NITE BP-03775 as of October 20, 2022 under the Budapest Treaty.

The NSK6 strain of *Hormonema macrosporum* has been internationally deposited with Patent Microorganisms Depositary, National Institute of Technology and Evaluation (Address: Room 122, 2-5-8 Kazusakamatari, Kisarazu-shi, Chiba, 292-0818 Japan) under the accession No. NITE BP-03774 as of October 20, 2022 under the Budapest Treaty.

The above-described fungal strains of *Hormonema macrosporum* in the present description include not only the strains themselves that have been deposited or registered in the predetermined institution under those microorganism names (hereinafter also referred to as "deposited strains" for the convenience of description), but also strains that are substantially equivalent thereto (also referred to as "derived strains" or "induced strains"). For the microorganisms, the "strains that are substantially equivalent" to the deposited strains means strains belonging to the same species as the deposited strains, having an identity of not less than 99.0%, not less than 99.5%, or 100% to the deposited strains in terms of the similarity of the genome sequence (the average nucleotide identity value), and in addition, having the same mycological properties as the deposited strains. The higher the similarity of the genome sequence to the deposited strains, the less likely undesired traits are to be expressed. For the microorganisms, the strains that are substantially equivalent to the deposited strains may be, for example, derived strains whose parent strains are the deposited strains. Examples of the derived strains include strains obtained by breeding from the deposited strains, and strains that have naturally occurred from the deposited strains. Examples of the breeding method include modification by a genetic engineering method, and modification by mutagenesis. Examples of the mutagenesis include X-ray irradiation; ultraviolet irradiation; and treatment with a mutagen such as N-methyl-N'-nitro-N-nitrosoguanidine, ethyl methane sulfonate, or methyl methane sulfonate. Examples of the strains that have naturally occurred from the deposited strains include strains that have naturally occurred during use of the deposited strains. Examples of such strains include mutant strains that have naturally occurred by culture (for example, subculture) of the deposited strains. The derived strains may be constructed by a single type of modification, or may be constructed by two or more types of modifications.

The extract of the cultured fungal cells of the fungus of the present disclosure comprises MYCs (which may hereinafter be referred to as "composition of the present disclosure").

The MYCs indispensably comprise MGn, and its content in the entire composition may be not less than 1% by weight, not less than 3% by weight, or not less than 5% by weight.

The MYCs may also comprise one, or two or more, selected from MGGnol, MGGcol, and MGa. In cases where the MYCs comprise not only MGn, but also one, or two or more, selected from MGGnol, MGGcol, and MGa, a higher anti-ultraviolet effect can be expected.

In cases where the composition of the present disclosure comprises MGGnol, its content in the entire composition may be not less than 30% by weight.

In cases where the composition of the present disclosure comprises MGGcol, its content in the entire composition may be not less than 0.5% by weight.

In general, MYCs have their ultraviolet-absorbing capacity due to structures containing cyclohexanone as a central ring. Therefore, confirmation of the presence of MYCs in an extract, and measurement of the amount and concentration of MYCs in an extract can be carried out using a light wavelength within the ultraviolet region. For example, these can be carried out by measuring the ultraviolet absorption of MYCs in the extract using a spectrophotometer (Patent Document 1, Non-Patent Document 2). The amount of MGGnol can be calculated by measuring the absorbance (ABS) at its maximum absorption wavelength, 310 nm, and performing calculation based on the molar absorption coefficient, 25,000 M⁻¹ cm⁻¹ (Patent Document 1). The amount of MGGcol can be calculated by measuring the absorbance at its maximum absorption wavelength, 310 nm, and performing calculation based on the molar absorption coefficient, 25,000 M⁻¹ cm⁻¹ (Non-Patent Document 5). The amount of MGa can be calculated by measuring the absorbance at its maximum absorption wavelength, 311 nm, and performing calculation based on the molar absorption coefficient, 20900 M⁻¹ cm⁻¹ (Non-Patent Document 5). Although MGn has been reported to have a maximum absorption wavelength of 310 nm (Non-Patent Document 5), its molar absorption coefficient has not been reported. The amount of MGn can be calculated by using instead the molar absorption coefficient of MGa, which shows almost the same molecular weight and has a similar structure.

The composition of the present disclosure may also comprise MYCs other than the above-described MYCs, and even other MAAs. Examples of the other MAAs, including the other MYCs, may include compounds containing cyclohexanone or cyclohexenimine as a central ring; or compounds in which various functional groups such as amino acids are bound to the central ring.

More specific examples thereof include mycosporine-glutaminol, mycosporine-glutamicol, mycosporine-hydroxyglutamicol, gadusol, deoxygadusol, mycosporine-glycine, mycosporine-taurine, mycosporine-alanine, mycosporine-serine, mycosporine-serinol, mycosporine-2-glycine, palythinol, palythenic acid, mycosporine-methylamine-threonine, mycosporine-glycine-valine, palythine, asterina-330, shinorine, porphyra-334, euhalothece-362, mycosporine-ornithine, mycosporine-lysine, mycosporine-glutamic acid-glycine, mycosporine-methylamine-serine, mycosporine-taurine, palythene, palythine-serine, palythine-serine-sulfate, and usujirene.

The MYC-containing composition of the present disclosure may be included in various compositions together with optional components. The route of administration (ingestion) of each composition is not limited, and may be, for example, transdermal administration, oral administration, ocular instillation, or nasal instillation. Examples of the mode of the composition include pharmaceuticals, quasi-drugs, foods and beverages (such as foods with function claims, and supplements), and cosmetics. In particular, taking the advantage of its ultraviolet-absorbing capacity, the composition may be included in sunscreen cosmetics. In one possible mode, the MYC-containing composition of the present disclosure itself may be a product such as a cosmetic.

The composition of the present disclosure may be produced by any method from a fungus that produces MGn.

Examples of a mode that may be implemented include a method by which MYCs can be extracted from *Hormonema macrosporum* to produce a MYC-containing fungal cell extract, the method specifically comprising: a culture step of culturing *Hormonema macrosporum;* and an extraction step of extracting an MYC fraction from cultured fungal cells of the fungus (which may be hereinafter referred to as the "production method of the present disclosure"). The *Hormonema macrosporum* to be used as the extraction source may be selected from the NSK102 strain (NITE BP-03776), the NSK33 strain (NITE BP-03775), and the NSK6 strain (NITE BP-03774) as described above.

The medium used in the culture step of culturing *Hormonema macrosporum* is not limited as long as the medium sufficiently allows the growth of the fungal strain, and may contain a carbon source, a nitrogen source, an inorganic salt, and, when necessary, components such as amino acids suitable for the growth of the microorganism. Examples of the carbon sources that may be used include sugars such as glucose, starch, and waste molasses; organic acids; and sodium acetate; especially sugars such as glucose. Examples of the nitrogen sources that may be used include ammonium salts, yeast extract, corn steep liquor, and peptone. Examples of the inorganic salts that may be used include magnesium salts, calcium salts, phosphate salts, iron salts, and copper salts. Usually, a liquid medium prepared by mixing and dissolving these components in water may be used.

The pH may usually be within the range of 2 to 9, 3 to 8, or 3.5 to 7.5. The growth of the fungus may be inhibited in cases where the pH is too high or too low.

The medium used in the culture may be a medium in a state where it does not substantially contain sodium ascorbate and/or peptone. The medium that "does not substantially contain" sodium ascorbate and/or peptone, used herein may be a medium in which the content of sodium ascorbate and/or peptone is not more than 0.1% by weight, not more than 0.01% by weight, or not more than 0.001% by weight, or is 0% by weight.

The amount of MYCs produced increases in *Hormonema macrosporum* cultured in a medium that does not substantially contain sodium ascorbate and/or peptone. Therefore, the amount of MYCs extracted from fungal cells of such a fungus can be improved.

The composition of the medium that does not substantially contain sodium ascorbate and/or peptone is not limited. Examples of the composition include a composition prepared by mixing glucose and yeast extract in water. Such a medium is suitable for large-scale culture of fungal cells and large-scale production of MYCs since the viscosity of the culture liquid can be kept low by using the medium.

In the culture step, the culture temperature is not limited as long as it is a condition that sufficiently allows the growth of the fungal cells. The culture temperature may be, for example, 15 to 40°C, 18 to 35°C, or 20 to 30°C. In cases where the culture temperature is too low, the growth rate is insufficient, while in cases where the culture temperature is too high, the biosynthetic pathways tend to be abnormal.

In the culture step, the culture period is not limited. The culture may be carried out for 72 hours to 240 hours, or 96 hours to 168 hours.

Further, in the culture step, light irradiation may or may not be carried out. Since *Hormonema macrosporum* can produce MYCs even without light irradiation, the culture cost can be reduced by performing the culture without light irradiation.

After the culture step, an extraction step of extracting an MYC fraction from the cultured fungal cells of *Hormonema macrosporum* into a solvent is carried out.

Here, in the extraction, the efficiency of extraction tends to be improved when the following (a) and/or (b) is/are satisfied. However, in cases where the following (c) is satisfied in the culture step, efficient extraction is possible even without carrying out a step satisfying (a) or (b). In other words, the production method of the present disclosure may satisfy one or more selected from the following (a), (b), and (c).
(a) In the extraction step, an MYC fraction is extracted into a solvent at not less than 40°C.
(b) Before the extraction step, a heating step of collecting fungal cells from the culture liquid after the culture step, and heating the collected fungal cells, is carried out.
(c) In the culture step, the culture is carried out in a medium containing glucose and yeast extract, and/or carried out without light irradiation.

After the culture step, but before the extraction step, fungal cells may be collected from the culture liquid. The collection of the fungal cells may be carried out by centrifugation, filtration, or the like. The collected fungal cells may be dried using a heat dryer, a freeze dryer, a vacuum dryer, a flash drier, or the like.

In the method of the present disclosure, in the (a) extraction of the MYC fraction into the solvent at not less than 40°C, the fungal cells are brought into contact with the extraction solvent.

As the extraction solvent, a polar solvent may be used, or a polar solvent containing mainly water may be used. Examples of such a polar solvent include water; and polar organic solvents such as lower alcohols (including methanol, ethanol, butyl alcohol, isopropanol, and butylene glycol) and acetone. Examples of the polar solvent containing mainly water include water; and aqueous solutions of a polar organic solvent such as a lower alcohol (for example, methanol, ethanol, butyl alcohol, isopropanol, or butylene glycol) or acetone. Water alone may also be used. As the solvent "containing mainly water", a solvent containing water at not less than 80% by weight, not less than 85% by weight, or not less than 90% by weight in the entire extraction solvent may be used. The higher the water content in the extraction solvent, the higher the efficiency of extraction can be expected to be. In other words, in cases where an aqueous solution of a polar organic solvent such as a lower alcohol is used as the extraction solvent, the concentration of the polar organic solvent may be not more than 20% by weight, or the organic solvent may not be substantially used. Here, "the organic solvent is not substantially used" means that the concentration of the polar organic solvent in the aqueous solution used as the extraction solvent is not more than 1% by weight, not more than 0.1% by weight, or not more than 0.01% by weight. In cases where the polar organic solvent is not substantially used in the extraction, the step of evaporating the polar organic solvent from the obtained extract can be eliminated.

The temperature of the extraction solvent in the extraction of the MYC fraction into the solvent is 40°C to 95°C, or may be 50°C to 90°C, or 60°C to 85°C. In cases where the temperature of the extraction solvent is too low, the extraction efficiency tends to decrease. In cases where the temperature of the extraction solvent is too high, a problem tends to occur in the stability of a desired component. Further, an apparatus such as an autoclave may be used to perform heating to not less than 90°C, or to a temperature of 100°C to 135°C, 110°C to 130°C, or 120°C to 125°C. The heating can inhibit the actions of undesired enzymes contained in the cells. In cases where the heating temperature is too low, the inhibition of the actions of undesired enzymes may be insufficient, while in cases where the heating temperature is too high, a problem tends to occur in the stability of a desired component. Further, pressurization may be carried out to not less than 0.10 MPa, not less than 0.12 MPa, not less than 0.15 MPa, or not less than 0.20 MPa. The pressurization promotes the inhibition of the actions of undesired enzymes contained in the cells. In cases where the pressurization is insufficient, the inhibition of the actions of undesired enzymes contained in the cells may be insufficient. From the viewpoint of safety, the pressurization may be carried out at not more than 1.00 MPa.

The extraction time may be 15 minutes to 120 minutes, 60 minutes to 110 minutes, or 90 minutes to 100 minutes. The longer the extraction time, the more likely the extracted amount is to be increased. The upper limit of the extraction time may be, for example, about 120 minutes. In cases where the extraction time is too long, undesired impurities are likely to be contained. During the extraction step, the fungal cells and the extraction solvent that have been brought into contact with each other may be stirred or shaken as appropriate, or may be left to stand.

After the culture step, the culture liquid may be subjected, directly or after being diluted as appropriate, to the extraction step without collecting the fungal cells from the culture liquid, to carry out the (a) extraction of the MYC fraction into the solvent at not less than 40°C. In this case, the culture liquid may be heated to not less than 40°C or not less than 50°C. Efficient extraction can be expected by the heating of the culture liquid in advance. Usually, the culture liquid contains a polar solvent, especially water.

The culture liquid after the culture step may be diluted, for example, not less than 2-fold by volume with water, a buffer, a medium, or the like before being subjected to the extraction step. In cases where the culture liquid is diluted with water, a buffer, a medium, or the like before its use in the extraction step, the culture liquid can be more easily handled. For example, in cases where the viscosity of the culture liquid is high, the culture liquid may be diluted with the buffer, medium, or the like before its use in the extraction step. By this, retention of the culture liquid in the apparatus can be reduced in some cases. The culture liquid may also be used directly in the extraction step. By the direct use of the culture liquid, the extraction cost may be reduced.

In the method of the present disclosure, the (b) heating step of heating, before the extraction step, the fungal cells collected from the culture liquid after the culture step may be carried out instead of or prior to the (a). In this step, under the following conditions, the fungal cells can be damaged or broken to improve the extraction efficiency of MYCs, or the actions of undesired enzymes in the fungal cells can be inhibited to improve the amount of MYCs recovered.

The heating of the fungal cells in the heating step may be carried out under conditions selected from the following in terms of the (i) temperature, (ii) length of time, and (iii) pressure.
(i) The temperature to which the fungal cells are exposed may be 40°C to 200°C, 50°C to 190°C, 60°C to 180°C, 70°C to 170°C, 80°C to 160°C, 90°C to 150°C, 100°C to 140°C, 105°C to 130°C, 110°C to 130°C, 115°C to 130°C, 120°C to 130°C, or 125°C to 130°C. In cases where the temperature to which the fungal cells are exposed is too low, the inhibition of the actions of undesired enzymes may be insufficient, while in cases where the temperature to which the fungal cells are exposed is too high, a problem tends to occur in the stability of a desired component.
(ii) The length of time for which the fungal cells are heated may be 2 to 6 hours, 3 to 5 hours, or 4 to 5 hours. The heating time may be a time for which a certain heating temperature is maintained after the temperature is achieved. In cases where the length of time for which the fungal cells are heated is too short, the inhibition of the actions of undesired enzymes may be insufficient, while in cases where the length of time for which the fungal cells are heated is too long, a problem tends to occur in the stability of a desired component.
(iii) The pressure applied to the fungal cells, calculated as the average pressure during the period when the fungal cells are exposed to heating, may be 0.01 MPa to 0.10 MPa, 0.02 MPa to 0.08 MPa, or 0.03 MPa to 0.05 MPa. In cases where the pressure applied to the fungal cells is too low, the inhibition of the actions of undesired enzymes may be insufficient, while in cases where the pressure applied to the fungal cells is too high, a problem tends to occur in the stability of a desired component.

The conditions (i) to (iii) may be arbitrarily selected and combined. In particular, at least (i) and (ii) may be combined. In this case, by heating at a high temperature for a certain length of time, sterilization can be achieved at the same time so that enzymes in the fungal cells can be inactivated. Therefore, the amount of MYCs recovered can be expected to be further improved. In addition, since the fungal cells are broken by (iii), the extraction efficiency of MYCs may be further improved. The heating step may be carried out under conditions where the fungal cells are dried. For example, the heating step may be carried out under one or more conditions selected from (i) a temperature of not less than 105°C, (ii) a heating time of not less than 2 hours, and (iii) a pressure of not more than 0.10 MPa. The heating step may also be carried out under the conditions of (i) a temperature of not less than 105°C, (ii) a heating time of not less than 2 hours, and (iii) a pressure of not more than 0.10 MPa.

In the production method of the present disclosure, in cases where the extraction step is carried out using fungal cells that have been subjected to the (b) heating step, the extraction step may be carried out under the conditions described in (a), or may be carried out at a low extraction temperature.

Specifically, the extraction solvent used may be the same as that described in (a). On the other hand, the temperature of the extraction solvent during the extraction of the MYC fraction into the solvent may be not less than 5°C, may be not less than 10°C, or may be not less than 25°C (normal temperature). The conditions described in (a) are also applicable. Thus, the temperature may be 40°C to 70°C, 50°C to 90°C, or 60°C to 100°C, or an apparatus such as an autoclave may be used to perform heating to a temperature of 90°C to 150°C, 100°C to 140°C, 110°C to 130°C, or 120°C to 125°C.

The extraction time may be not less than 15 minutes, not less than 60 minutes, or not less than 90 minutes. The longer the extraction time, the more likely the extracted amount is to be increased. The upper limit of the extraction time may be, for example, about 120 minutes. During the extraction step, the fungal cells and the extraction solvent that have been brought into contact with each other may be stirred, may be shaken, or may be left to stand as appropriate.

By exposing the fungal cells, and/or the culture liquid containing the fungal cells to a high temperature condition during or before the extraction step as described above, the fungal cells can be killed, and/or enzymes having an action to degrade MYCs in the fungal cells can be inactivated, so that the amount of MYCs recovered can be improved. The high temperature condition to which the culture liquid is exposed may be, for example, not less than 40°C or not less than 50°C.

Therefore, in the production method of the present disclosure, the survival rate of the fungal cells after the extraction step may be 0% to 20%, 0% to 10%, or 0% to 5% compared to those cells after the culture step. The survival rate is not limited as long as the survival rate after the extraction step is within this range. However, in cases where the survival rate of the fungal cells is low also before the extraction step or during the extraction step, degradation of MYCs can be expected to be suppressed to improve the amount of MYCs recovered.

After the extraction step, the obtained extract may be subjected to removal of foreign substances, fractionation, purification, and the like by well-known methods.

The MYCs, including MGn, MGGnol, MGGcol, and MGa, are water-soluble. Therefore, even under high temperature conditions, they are not lost due to degradation or the like, and can be obtained in a sufficient amount from cultured fungal cells of *Hormonema macrosporum.* Conventionally, it has been expected that the ultraviolet-absorbing ring structures of MAAs including MYCs may be easily degraded by heat, and hence that MAAs can be hardly obtained in cases where they have undergone heat treatment. Therefore, those skilled in the art have not expected the fact that the production method of the present disclosure enables maintenance of the ultraviolet-absorbing capacity and recovering of MYCs without a decrease in their amount, to allow production of a composition containing not less than a certain amount of MGn.

Unless otherwise defined, all technical terms and scientific terms used herein have the same meanings as those generally understood by those skilled in the art to which the present disclosure belongs. Any of the methods and materials similar or equivalent to the methods and materials described herein can be used in the practice or testing of the present disclosure, and representative exemplary methods and materials are described herein.

It is understood that, when a range of values is provided, the individual values between the upper limit and the lower limit of the range (to the tenth of the unit of the lower limit unless the context clearly dictates otherwise), and any other described values or intervening values within the described range are encompassed within the scope of the invention presented in the present disclosure. The upper and lower limits of a narrower range thereof may be independently included in the narrower range. They are also encompassed within the scope of the invention presented in the present disclosure, and subject to any specifically excluded limit in the described range. In cases where a described range includes one or both of the limits, ranges excluding one or both of those included limits are also included in the invention presented in the present disclosure.

This present disclosure is not limited to the specific modes described. Since the scope of the invention presented in the present disclosure is considered to be limited only by the appended claims, it should also be understood that the terminology used herein is merely for the purpose of describing particular modes, and is not intended to be limiting.

As will be apparent to those skilled in the art upon reading this disclosure, each of the individual modes described herein has discrete components and features that may be readily separated from or combined with features of several other modes without departing from the scope and the spirit of the invention of the present disclosure. Any cited method may be implemented in the order of the cited events or in any other order that is logically possible and consistent.

All publications and patents cited herein are hereby incorporated by reference as if each individual publication or patent were specifically and individually indicated to be incorporated by reference, and the publications are incorporated herein by reference to disclose and describe the methods and/or materials with which the publications are cited in connection. The citation of any publication is for its disclosure prior to the filing date, and should not be construed as an admission that, by virtue of prior invention, the invention described in the present disclosure is not entitled to antedate such publication. Further, the dates of publication provided may be different from the actual publication dates, and the actual publication dates may need to be individually confirmed.

The following Examples are given for the purpose of exemplifying various modes of the present disclosure, and are not intended to limit the present disclosure in any fashion. Those skilled in the art will readily appreciate that the present disclosure is well adapted to carry out the mentioned objects to achieve the final goal and the advantages mentioned, and also well adapted to carry out the inherent objects of the present description to achieve the final goal and the advantages. The present Examples, along with the methods described herein, are representative modes at present. They are exemplary, and not intended to limit the scope of the present disclosure. Modifications and other uses encompassed within the spirit of the present disclosure as defined by the claims will occur to those skilled in the art.

### EXAMPLES

The invention presented in the present disclosure is described below by way of Examples, but the invention presented in the present disclosure is not limited by these Examples. Unless otherwise specified, the units are on a weight basis.

### (1) Culture

A liquid medium was prepared by mixing 3% by weight glucose (manufactured by FUJIFILM Wako Pure Chemical Co., Ltd., special-grade reagent) and 1% by weight yeast extract (manufactured by TASTONE 154AG SENSIENT) in water. After placing 100 mL of the liquid medium in a 500-mL Erlenmeyer flask, heat sterilization was carried out at 121°C for 15 minutes.

*Aureobasidium pullulans* (the 6353 strain, the 7757 strain, the 100716 strain, the 106987 strain, the 114573 strain, and 108784) obtained from NBRC, *Sydowia polyspora* (the 107026 strain, the 107027 strain, and the 107028 strain) obtained from NBRC, and *Hormonema macrosporum* (the NSK102 strain, the NSK33 strain, and the NSK6 strain) separated from natural sources were inoculated to PDA plates (potato dextrose agar medium, granular (manufactured by Nissui Pharmaceutical Co., Ltd.)), and cultured at 25°C for 4 days, followed by storage at 4°C. A loopful of grown fungal cells were inoculated to the sterilized liquid medium, and shake culture was performed at 25°C at 100 rpm for 7 days.

The entire culture liquid after the culture was centrifuged at 8000 rpm for 5 minutes to collect the fungal cells separated from the supernatant. The fungal cells were then lyophilized. The dry fungal cell weight was measured to calculate the amount of fungal cells per 1 L of the culture liquid.

### (2) Extraction

After taking 20 mg ± 2 mg of the collected fungal cells into a lidded test tube, 3 mL of water was added thereto, and the fungal cells were suspended with stirring. The test tube was then heated to 80°C on a block heater, and heat treatment was carried out for 60 minutes with stirring by vortexing at 15-minute intervals. After the test tube was left to stand to allow the fungal cells to precipitate, 1 mL of the supernatant was dispensed into a centrifuge tube, and centrifugation was carried out at 12,000 rpm for 3 minutes, followed by collecting the resulting supernatant, to provide an HPLC measurement sample.

### (3) Analysis of MAAs

### (3-1) Liquid Chromatography

MAAs contained in the fungal cell extract were analyzed by HPLC with reference to Non-Patent Document 1. Specifically, an HPLC system manufactured by Shimadzu Corporation was used with the following: a Lichrospher (registered trademark) 100RP-18 column (particle size, 5 µm; 4mm (inner diameter) × 250 mm (length); manufactured by Merck) equipped with a guard column LiChrospher (registered trademark) 100 RP-18 (5 µm) LiChroCART (registered trademark) 4-4; a mobile phase (aqueous solution of 0.5% methanol and 0.2% acetic acid); a flow rate of 0.7 mL/min.; a column oven temperature of 35°C; an injection volume of 10 µL; and measurement at 250 nm to 400 nm using a PDA inspection device. A peak with a maximum absorption wavelength near 310 nm was found, and this was regarded as the peak of MAAs. Quantification was carried out at a detection wavelength of 310 nm.

### (3-2) Peak Identification by LC-MS

The fungal cell extract was analyzed using an HPLC/MS system manufactured by Agilent Technologies, Inc. under the same conditions as in (3-1), and the molecular weight of each peak detected at 310 nm was determined. As a result, the peaks were identified as shown in Table 1. The MAAs identified were MGn, MGGnol, MGa, and MGGcol, all of which were mycosporines. Therefore, they were defined as mycosporines (MYCs) as a whole, and the total content of the four types was defined as the amount of MYCs.

### [Table 1]

**Table 1**

| MAAs | Abbreviation | HPLC RT (min) | Mass |
|---|---|---|---|
| Mycosporine-glutamine | MG n | 6.5 | 316.1 |
| Mycosporine-glutaminol-glucoside | MGGnol | 8 | 464.2 |
| Mycosporoine-glutamic acid | MGa | 10.8 | 317.1 |
| Mycosporine-glutamicol-glucoside | MGGcol | 12.5 | 465.2 |

### (3-3) Analysis of MYC Concentration by Liquid Chromatography

As a result of HPLC analysis of the fungal cell extract of the *Aureobasidium pullulans* NBRC100716 strain, only the peak of MGGnol could be detected. The sample was subjected to wavelength scanning within the range of 400 nm to 250 nm using a spectrophotometer. As a result, only a peak at 310 nm was detected. The sample was diluted with water such that the absorbance at 310 nm was 1 (Sample A). The resulting dilution was further diluted 1-fold, 2-fold, 3-fold, and 5-fold with water, and the peak area at 310 nm was measured by HPLC for each aqueous solution. Since the molar absorption coefficient of MGGnol is 25,000 M⁻¹ cm⁻¹ (Patent Document 1), the MGGnol concentration in an aqueous solution with an absorbance of 1 is 118.7 µg/mL. From the obtained peak area and absorbance of MGGnol, a correlation equation for the MGGnol concentration in Sample A was provided.

The concentration was calculated also for each of the other MYCs based on the ratio of its molar absorption coefficient to that of MGGnol. MGGcol has been reported to have a molecular weight of 465, a maximum absorption wavelength of 310 nm, and a molar absorption coefficient of 25,000 M⁻¹ cm⁻¹ (Non-Patent Document 5). MGa has been reported to have a molecular weight of 317, a maximum absorption wavelength of 311 nm, and a molar absorption coefficient of 20,900 M⁻¹ cm⁻¹ (Non-Patent Document 5). Although MGn has been reported to have a molecular weight of 316 and a maximum absorption wavelength of 310 nm (Non-Patent Document 5), its molar absorption coefficient has not been reported. Therefore, the molar absorption coefficient of MGa, which shows almost the same molecular weight and has a similar structure, was used for MGn.

### (4) Results

Table 2 shows, for each fungal strain, the dry fungal cell weight, the MYC content (mg/g) (the amount of MYCs per 1 g of fungal cells) after 60 minutes of extraction with water at 80°C, the MYC yield (g/L) (the amount of MYCs per 1 L of the culture liquid, MGG content × dried fungal cells), the composition ratios (%) in the MAA composition, and the external appearance of the living fungal cells as observed under the microscope.

Although the cultures of the fungal cells of the fungi of Comparative Examples did not contain MGn at all, the cultures of the fungi of Examples (specific strains of *Hormonema macrosporum)* contained MGn, and also contained MGGnol, MGGcol, and MGa.

### [Table 2]

**Table 2**

| | | Fungal strain | Dry fungal cells (g/L) | MYCs content (mg/g) | MYCs yield (g/L) | MAAs composition (%) | | | | External appearance (microscopy) |
|---|---|---|---|---|---|---|---|---|---|---|
| | | | | | | MGn | MGGnol | MGa | MGGcol | |
| Comparative Example | *Aureobasidium pullulans* | NBRC6353 | 12.2 | 14.7 | 0.18 | 0 | 100 | 0 | 0 | Yeast type |
| | | NBRC7757 | 13.3 | 7.0 | 0.09 | 0 | 97 | a | 3 | Yeast type |
| | | NBRC100716 | 10.8 | 19.9 | 0.21 | 0 | 100 | 0 | 0 | Yeast type |
| | | NBRC106987 | 9.6 | 13.6 | 0.13 | 0 | 98 | 0 | 2 | Yeast type |
| | | NBRC114573 | 11.9 | 5.3 | 0.06 | 0 | 100 | 0 | 0 | Yeast type |
| | | NBRC108784 | 12.3 | 13.4 | 0.16 | 0 | 97 | 0 | 3 | Yeast type |
| | *Hormonema macrosporum (Sydowia polysopora)* | NBRC107026 | 12.4 | 6.4 | 0.08 | 0 | 95 | 0 | 5 | Yeast type |
| | | NBRC107027 | 13.8 | 4.7 | 0.06 | 0 | 96 | 0 | 4 | Yeast type |
| | | NBRC107028 | 14.6 | 5.0 | 0.07 | 0 | 90 | 0 | 11 | Yeast type |
| Example | *Hormonema macrosporum (Sydowia polysopora)* | NSK102 | 13.3 | 6.5 | 0.11 | 7 | 83 | 1 | 9 | Yeast type |
| | | NSK33 | 15.8 | 15.9 | 0.24 | 5 | 86 | 1 | 8 | Yeast type |
| | | NSK6 | 15.0 | 16.8 | 0.23 | 5 | 87 | 1 | 7 | Yeast type |

## Claims

1. An extract of cultured fungal cells of a fungus, the extract comprising a mycosporine (MYC),
the MYC comprising mycosporine-glutamine (MGn).

2. The extract according to claim 1, wherein the MYC further comprises one, or two or more, selected from mycosporine-glutaminol-glucoside (MGGnol), mycosporine-glutamicol-glucoside (MGGcol), and mycosporine-glutamic acid (MGa).

3. The extract according to claim 1 or 2, wherein the fungus is a fungus belonging to the genus *Hormonema.*

4. The extract according to claim 3, wherein the fungus belonging to the genus *Hormonema* is *Hormonema macrosporum.*

5. The extract according to claim 4, wherein the *Hormonema macrosporum* is selected from the NSK102 strain (NITE BP-03776), the NSK33 strain (NITE BP-03775), and the NSK6 strain (NITE BP-03774).

6. The composition according to claim 1, wherein MGn is contained at not less than 1% by weight in the entire composition.

7. The extract according to claim 2, comprising MGGnol at not less than 30% by weight in the entire composition.

8. The extract according to claim 2, comprising MGGcol at not less than 0.5% by weight in the entire composition.

9. The extract according to claim 1, which is a water extract.

10. A method of producing an MYC-containing fungal cell extract, the method comprising: a culture step of culturing *Hormonema macrosporum;* and an extraction step of extracting a mycosporine (MYC) fraction from cultured fungal cells of the *Hormonema macrosporum;*
wherein the *Hormonema macrosporum* is selected from the NSK102 strain (NITE BP-03776), the NSK33 strain (NITE BP-03775), and the NSK6 strain (NITE BP-03774).

11. The production method according to claim 10, wherein the extract is a polar solvent extract, the method satisfying one or more selected from the following (a), (b), and (c):
(a) in the extraction step, the MYC fraction is extracted into a polar solvent at not less than 40°C;
(b) before the extraction step, a heating step of collecting fungal cells from the culture liquid after the culture step, and heating the collected fungal cells, is carried out; and
(c) in the culture step, the culture is carried out in a medium containing glucose and yeast extract, and/or carried out without light irradiation.

12. The production method according to claim 11, wherein the (a) is carried out by heating the culture liquid after the culture step to not less than 50°C.

13. The production method according to claim 11, wherein the (b) heating step is carried out by heating under one or more conditions selected from the group consisting of (i) to (iii):
(i) a temperature of not less than 105°C;
(ii) a heating time of not less than 2 hours; and
(iii) a pressure of not more than 0.10 MPa.

14. The production method according to claim 10, wherein the MYC comprises: MGn; and one, or two or more, selected from MGGnol, MGGcol, and MGa.

15. The production method according to claim 11, wherein the polar solvent is water.

16. The production method according to claim 10, wherein ethanol and/or butyl alcohol is/are not substantially used as the extraction solvent.
